# EUROPÄISCHE PATENTSCHRIFT

(11) **EP 0 922 958 B1**
(45) Veröffentlichungstag und Bekanntmachung des Hinweises auf die Patenterteilung: **02.10.2002**
(21) Anmeldenummer: 98123351.3
(22) Anmeldetag: 08.12.1998
(51) Int. Cl.: G01N 33/53, G01N 33/543

(54) **Entstörung von diagnostischen Verfahren durch Peptide aus D-Aminosäuren**
Interference reduction in diagnostic methods using D-amino acid peptides
Réduction d'interférence dans les méthodes diagnostiques utilisants les peptides comportant les D-acides amines

(30) Priorität: 11.12.1997 DE 19755078; 24.04.1998 DE 19818383
(43) Veröffentlichungstag der Anmeldung: 16.06.1999
(73) Patentinhaber: Roche Diagnostics GmbH, 68298 Mannheim (DE)
(72) Erfinder: Seidel, Christoph Dr., 82362 Weilheim (DE); Lenz, Helmut Dr., 82327 Tutzing (DE); Karl, Johann Dr., 82380 Peissenberg (DE); Ofenloch-Hähnle, Beatus Dr., 82398 Polling (DE); Klause, Ursula Dr., 82380 Peissenberg (DE); Faatz, Elke Dr., 82386 Huglfing (DE)

(56) Entgegenhaltungen:
- EP-A- 0 331 068
- EP-A- 0 378 175
- EP-A- 0 525 916
- EP-A- 0 747 699
- WO-A-95/23801
- US-A- 4 810 635

## Beschreibung

Gegenstand der vorliegenden Erfindung ist die Verwendung von Peptiden, die im wesentlichen aus D-Aminosäuren bestehen, zur Entstörung von diagnostischen Verfahren, ein Verfahren zur Entstörung von diagnostischen Verfahren durch Peptide, die im wesentlichen aus D-Aminosäuren bestehen, sowie ein Entstörreagenz, enthaltend mindestens ein Peptid, das im wesentlichen aus D-Aminosäuren besteht.

In der Diagnostik haben in den vergangenen Jahren insbesondere immunologische Nachweisverfahren große Bedeutung erlangt. Durch diese Verfahren können Analyten in biologischen Proben nachgewiesen werden. Zu diesen Analyten zählen beispielsweise Arzneimittel, Hormone, Proteine, infektiöse Agenzien, Mikroorganismen und Antikörper gegen diese Analyten. Insbesondere zum Nachweis von Infektionen durch Mikroorganismen wie Bakterien, Pilze oder Viren werden diese Erreger entweder direkt oder indirekt nachgewiesen. Das heißt, daß je nach Infektion der Erreger durch den Antigennachweis diagnostiziert wird oder die Antikörper, die als Immunantwort spezifisch gegen die Erreger gebildet wurden, nachgewiesen werden.

Bei allen immunologischen Nachweisreaktionen kommt es zu einer spezifischen Bindungsreaktion zwischen der Substanz, die nachgewiesen werden soll (Analyt) und mindestens einem spezifischen Bindepartner, der mit dem Analyten spezifisch reagiert oder ihn spezifisch bindet. Der Analyt und der spezifische Bindepartner bilden dabei ein spezifisches Bindungspaar, im allgemeinen ein Komplex zwischen einem Antigen und einem Antikörper oder einem Antikörper-Fragment. Dabei können mehr als ein Analyt oder mehr als ein Bindepartner in jeder Reaktion miteinander reagieren. Die Detektion dieser spezifischen Bindereaktionen kann auf verschiedene Weise erfolgen. Im allgemeinen ist ein Bindepartner der spezifischen Bindereaktion markiert. Übliche Markierungen sind Chromogene, Fluorophore, zur Chemi- oder Elektrochemilumineszenz fähige Substanzen, Radioisotope, Haptene, Enzymmarkierungen oder Substanzen, die wiederum ein spezifisches Bindungspaar bilden können wie beispielsweise Biotin/Streptavidin.

Ein schwerwiegendes Problem bei Immunoassays ist, daß zwischen den spezifischen Bindepartnern des Immunoassays und den Probenbestandteilen unerwünschte Wechselwirkungen und unspezifische Bindereaktionen erfolgen können. Derartige Wechselwirkungen bewirken im allgemeinen eine Erhöhung des Hintergrundsignals, eine stärkere Streuung der Signale und damit eine verringerte Sensitivität und Spezifität des betreffenden Tests.

Je nach Art der durch unspezifische Wechselwirkungen hervorgerufenen Störung kann es auch zu falsch-positiven oder zu falsch-negativen Testergebnissen kommen.

Zu falsch-negativen Ergebnissen kann es dann kommen, wenn sich in der Probe eine Substanz befindet, die den nachzuweisenden Analyten maskiert, so daß die spezifischen Nachweisreagenzien, beispielsweise ein Antikörper, nicht mehr an den Analyten binden können.

Ein besonders großes Problem stellen falsch-positive Testergebnisse dar. Das heißt, daß trotz Abwesenheit des Analyten im Test ein positives Signal erhalten wird. So darf es insbesondere bei der Diagnostik von Infektionskrankheiten nicht vorkommen, daß Proben von gesunden, nichtinfizierten Patienten im Test ein falsch-positives Ergebnis liefern. Bei der Diagnostik von HIV-Infektionen sind beispielsweise die von den Zulassungsbehörden gestellten Anforderungen an die klinische Spezifität von diagnostischen Tests zum Nachweis von Anti-HIV-Antikörpern größer als 99,5 %. Das heißt, daß in einem Normalspenderkollektiv (Proben von Nichtinfizierten) von 1000 Proben nicht mehr als 5 falsch-positive Proben auftreten dürfen. Die dennoch auftretenden falsch-positiven Reaktionen werden verursacht durch unspezifische Substanzen, die je nach Testmethode an die zum Antikörper-Nachweis eingesetzten Antigene, beispielsweise HIV-Antigene, binden und dann wie Antikörper gegen das nachzuweisende infektiöse Agens mit Hilfe des Nachweissystems falsch-positiv detektiert werden. Oftmals handelt es sich bei diesen unspezifisch reagierenden Substanzen um Antikörper. Im Stand der Technik sind bereits verschiedene Versuche beschrieben, um diese unspezifischen Wechselwirkungen in Immunoassays zu reduzieren, die zu falschen Testergebnissen führen. So ist seit langem bekannt, daß unterschiedliche Kohlenhydrat-Komponenten und Proteine, Proteingemische, bestimmte Proteinfraktionen sowie deren Hydrolysate unspezifische Wechselwirkungen zwischend den Testkomponenten und dem Analyten im Immunoassays reduzieren können (siehe beispielsweise Robertson et al., J. Immunol. Meth. 26, 1985; EP-A-0 260 903; US 4,931,385). Der Einsatz von Protein-Rohfraktionen und Rohhydrolysaten hat den Nachteil, daß durch die darin enthaltenen Bestandteile wiederum andere Störungen des Test ausgelöst werden können. Enzymatisch produzierte Hydrolysate können zudem mit den bei der Herstellung verwendeten Proteasen kontaminiert sein und weisen in der Regel keine einheitliche Qualität auf, da sich die enzymatische Spaltung nur schwer steuern läßt. Protease-Kontaminationen können Testkomponenten angreifen und schon in geringen Mengen zur Beeinträchtigung der Testfunktionen und Lagerstabilität führen.

Zur Verringerung unspezifischer Wechselwirkungen in Immunoassays wurde auch der Einsatz von chemisch modifizierten Proteinen, insbesondere von succinylierten oder acetylierten Proteinen beschrieben (US 5,051,356; EP-A-0 525 916). Mit diesen Substanzen können jedoch viele der falsch-positiven Ergebnisse bei Antikörpernachweisen aus Serumproben nicht vermieden werden.

Die EP-A-0 331 068 und WO 91/06559 beschreiben den Einsatz von polymerisierten Immunglobulinen, insbesondere IgG, zur Reduktion spezifischer Störfaktoren wie z. B. Rheumafaktoren. Es lassen sich damit aber nicht alle störenden Wechselwirkungen zufriedenstellend ausschalten. Außerdem kann der Zusatz von unspezifischen humanen Immunglobulinen in Tests auf humane Antikörper zu einer Erhöhung des Leerwerts führen. Ferner ist die Gewinnung von humanem oder tierischem IgG aufwendig und teuer.

In der WO 95/23801 werden Avidin und Streptavidin sowie deren Derivate als Entstörmittel beschrieben, die in heterogenen Immunoassays vor allem unspezifische Wechselwirkungen der Probenbestandteile mit einer Streptavidin- oder Avidin-Festphase reduzieren. Eine Entstörung von Substanzen, die keine Wechselwirkungen mit der Festphase eingehen, sondern unspezifisch an die im allgemeinen immunologischen Testbestandteile binden, ist mit diesen Entstörmitteln nicht möglich.

Im Stand der Technik ist bisher keine zufriedenstellende Lösung für das Problem beschrieben worden. Aufgabe war es daher, neue Entstörsubstanzen zur Verfügung zu stellen, um eine bessere Entstörung von unspezifischen Wechselwirkungen bei Immunoassays zu bewirken als im Stand der Technik bisher bekannt ist. Insbesondere lag der Erfindung die Aufgabe zugrunde, falsche Nachweisergebnisse bei der Diagnose von Infektionskrankheiten zu verringern oder wenn möglich vollkommen zu vermeiden.

Die Aufgabe wird gelöst durch die Verwendung von Peptiden, die im wesentlichen aus D-Aminosäuren bestehen, zur Entstörung von immunologischen Nachweisverfahren. Es hat sich überraschenderweise gezeigt, daß durch den erfindungsgemäßen Einsatz von Peptiden, die im wesentlichen aus D-Aminosäuren bestehen, eine drastische Reduzierung von unspezifischen Wechselwirkungen und somit eine weitgehende Vermeidung falscher und insbesondere falsch-positiver Nachweisreaktionen möglich ist.

Eine wichtige Eigenschaft der als Entstörmittel eingesetzten Peptide ist, daß diese nicht aus den natürlicherweise vorkommenden L-Aminosäuren, sondern im wesentlichen aus D-Aminosäuren bestehen. Dadurch werden Eigenschaften wie Hydrophilie, Flexibilität und Löslichkeit exakt nachgeahmt, ohne daß die antigenen Eigenschaften denen der entsprechenden Peptide aus L-Aminosäuren entsprechen. Die Antigenität der Peptide, die im wesentlichen aus D-Aminosäuren bestehen, ist deshalb nicht vorhanden, da deren sterische Konformation sich im Vergleich mit den tatsächlichen Antigenen genau spiegelbildlich verhält. Unter Antigenität ist das spezifische Bindungsverhalten von zwei oder mehreren immunologischen Bindepartnern wie beispielsweise von einem Antigen und einem spezifischen Antikörper zueinander zu verstehen. Somit passen die Peptide aus D-Aminosäuren nicht mehr in die Antigenbindungsstelle, d.h. das Paratop der nachzuweisenden spezifischen Antikörper. Das erfindungsgemäße Entstörmittel behindert somit die spezifische Nachweisreaktion nicht.

Unter D- und L-Aminosäuren sind die Projektionsformeln von Aminosäuren gemäß der Nomenklatur von Emil Fischer für optisch aktive (chirale) Verbindungen zu verstehen. Danach wird die Kohlenstoffkette senkrecht so angeordnet, daß das Ende mit der höchsten Oxidationsstufe oben liegt. Das Molekül muß zudem so angeordnet werden, daß am asymmetrischen C-Atom die Substituenten nach vorn ragen. Zur Bezeichnung der Konfiguration bei Aminosäuren bezieht man sich auf die Aminogruppe, d.h. L-Konfiguration bei links stehender Aminogruppe und D-Konfiguration bei rechts stehender Aminogruppe. Die Bezeichnungen D und L haben jedoch nichts mit der Richtung der optischen Drehung von linear polarisiertem Licht zu tun. Die D- und die L-Form einer Aminosäure verhalten sich zueinander wie Bild und Spiegelbild. Proteine bestehen ausschließlich aus L-Aminosäuren. Lediglich einige Bakterien besitzen in ihrer Zellwand D-Aminosäuren.

Unter dem Begriff "Peptid" werden aus mindestens 2, maximal aus 100 Aminosäuren aufgebaute, über Amidbindungen miteinander kovalent verknüpfte Moleküle verstanden. Diese Definition gilt sowohl für Peptide aus L- als auch aus D-Aminosäuren.

In Immunoassays werden oftmals Peptide aus L-Aminosäuren als immunologische Bindepartner eingesetzt. Diese als Nachweisreagenzien verwendeten Peptide können, müssen aber nicht mit anderen Molekülen markiert sein. Zu diesen Markierungen zählen beispielsweise Biotin, Haptene wie Digoxigenin oder Steroidhormone, Enzyme, Farbstoffe, Fluoreszenzmarkierungen. Als immunologische Bindepartner verwendete Peptide aus L-Aminosäuren binden in Immunoassays spezifisch an einen anderen Bindepartner, wie zum Beispiel an den Analyten, der in diesem Fall ein Antikörper sein kann. Wird ein markiertes Peptid verwendet, so kann die Anwesenheit des Analyten durch die Markierung des an den Analyten gebundenen Peptids nachgewiesen werden.

Die Peptide aus L-Aminosäuren können auch als Kompetitoren eingesetzt werden. Hierbei verdrängt das Peptid aus L-Aminosäuren einen der immunologischen Bindepartner, beispielsweise eines Antigen-Antikörper-Komplexes, aus dem Komplex. Anhand der Menge der entweder im Komplex oder in der Lösung befindlichen markierten Peptids kann durch Vergleich mit einer Standardkurve auf die Konzentration des Analyten geschlossen werden.

Die Sequenz der als immunologische Bindepartner oder Nachweisreagenzien eingesetzten Peptide aus L-Aminosäuren orientiert sich entweder an der Sequenz des nachzuweisenden Analyten oder an der Sequenz eines immunologischen Bindepartners, der den Analyten spezifisch bindet. Als Sequenzbereich wird dabei im allgemeinen ein definierter Epitopbereich ausgewählt, von dem bekannt ist, daß er immunologisch erkannt wird. Insbesondere auf dem Gebiet der Infektionskrankheiten wie HIV- und HCV-Infektionen sind bereits eine Reihe von immunologisch aktiven Epitopen der Virusproteine beschrieben worden. Zu den wichtigsten immunologisch aktiven Bereichen von HIV zählen die Envelope (env)-Proteine gp41, gp120 und das Capsid (gag)-Protein p24.

Bei der Diagnostik von Infektionskrankheiten werden jedoch auch oftmals vollständige Proteine oder deren Fragmente als immunologische Bindepartner eingesetzt, die entweder aus ihrer nativen Quelle aufgereinigt oder rekombinant hergestellt werden.

Die Aminosäuresequenz der erfindungsgemäß zur Entstörung eingesetzten Peptide, die im wesentlichen aus D-Aminosäuren bestehen, orientiert sich - analog zu den Peptiden aus L-Aminosäuren - an der Sequenz, die entweder auf dem Analyten selbst oder auf einem zum Analyt-Nachweis eingesetzten analyt-spezifischen Bindepartner vorhanden ist. Bevorzugt entspricht die Sequenz des zur Entstörung eingesetzten Peptides dem Epitop auf dem Analyten oder dem Epitop auf dem spezifischen Bindepartner.

Werden als spezifische Bindepartner für die Immunreaktion L-Peptide eingesetzt, so wird bei der Auswahl der zur Entstörung eingesetzten Peptide bevorzugt ein Peptid gleicher Länge und gleicher Sequenz, das vollständig aus D-Aminosäuren besteht, eingesetzt.

Die Länge der erfindungsgemäßen Peptide, die im wesentlichen aus D-Aminosäuren bestehen, beträgt mindestens 4 bis 50, bevorzugt 5 bis 20 D-Aminosäuren.

Wesentlich an den erfindungsgemäßen Peptiden ist, daß sie nicht aus L-, sondern im wesentlichen aus D-Aminosäuren bestehen. Dabei ist die Verwendung von Glycin, das kein asymmetrisches C-Atom besitzt und damit weder D- noch L-konfiguriert ist, ausdrücklich erlaubt. Unter der Bezeichnung "im wesentlichen aus D-Aminosäuren bestehend" ist ein Peptid zu verstehen, dessen Sequenz, die dem Epitop auf dem Analyten oder dem spezifischen Bindepartner entspricht, aus D-Aminosäuren besteht. Es ist jedoch im Rahmen der Erfindung möglich, daß dieser Epitopbereich von Aminosäuren flankiert wird, die die natürliche L-Konfiguration besitzen. Dies kann dann erforderlich sein, wenn Spacerbereiche aus L-Aminosäuren an den Epitopbereich gekoppelt werden. Es können jedoch auch andere chemische Gruppen wie beispielsweise Alkylketten als Spacer oder Abstandshalter verwendet werden. Diese Spacer werden oftmals benötigt, um andere Moleküle an das Entstörpeptid zu koppeln. Wichtig ist dabei jedoch, daß das für die Entstörung relevante Epitop aus D-Aminosäuren aufgebaut ist. Es ist erfindungsgemäß auch möglich, daß die flankierenden Bereiche aus Lipidstrukturen, wie sie beispielsweise in Phospholipiden oder Lipoproteinen vorkommen, oder aus Zuckerketten bestehen. Die das Epitop flankierenden Bereiche des Entstörpeptids können prinzipiell beliebig modifiziert werden. Wichtig bei allen Peptid-Modifikationen ist, daß die Entstörwirkung dadurch nicht beeinträchtigt wird. Auch muß darauf geachtet werden, daß das zur Entstörung eingesetzte Peptid aufgrund von zusätzlichen Modifikationen selbst keine Störung des Immunoassays hervorruft.

Die weiter oben beschriebene Länge der erfindungsgemäßen Peptide bezieht sich auf das aus D-Aminosäuren aufgebaute Epitop. Es ist durchaus möglich, daß die Gesamtlänge des Entstörpeptids durch den Einbau flankierender Sequenzen über die Obergrenze von 50 Aminosäuren hinausgeht.

Die Herstellung der Peptide, die im wesentlichen aus D-Aminosäuren bestehen, erfolgt durch chemische Synthese an einer Festphase, vorzugsweise mit einem kommerziellen Peptidsynthesegerät. Bevorzugt wird die Herstellung nach der dem Fachmann geläufigen Methode nach Merrifield durchgeführt. Die einzelnen Aminosäure-Bausteine müssen dabei dort, wo es erforderlich ist, als D-Isomer eingesetzt werden. Die übrigen Verfahrensschritte entsprechen der Synthese von Peptiden aus L-Aminosäuren. Das heißt, die Synthese erfolgt nach bekannten Methoden, vorzugsweise ausgehend vom Carboxylterminus des Peptids unter Verwendung von Aminosäurederivaten (hier: D-Aminosäurederivate). Vorzugsweise werden Aminosäurederivate eingesetzt, deren für die Kopplung benötigte Amino-Endgruppe mit einem Fluorenymethyloxycarbonyl (Fmoc)-Rest derivatisiert ist. Reaktive Seitengruppen der eingesetzten Aminosäuren enthalten Schutzgruppen, die nach Beendigung der Peptidsynthese ohne weiteres abspaltbar sind. Bevorzugte Beispiele hierfür sind Schutzgruppen wie etwa Triphenylmethyl (Trt), t-Butylether (tBu), t-Butylester (OtBu), ter.-Butyloxycarbonyl (Boc) oder 2,2,5,7,8-Pentamethylchroman-6-sulfonyl (Pmc).

Für die Peptidsynthese können die 19 Standard-Aminosäuren in D-Form eingesetzt werden, die natürlicherweise in L-Form vorkommen. Darüberhinaus können auch die D-Formen von Aminosäurederivaten wie beispielsweise 4-Hydroxyprolin, 5-Hydroxylysin, 3-Methylhistidin, Homoserin oder Ornithin eingesetzt werden.

Nach Beendigung der Synthese erfolgt gegebenenfalls nach Freisetzung des Peptids von der Festphase eine Abspaltung von Schutzgruppen nach dem Fachmann geläufigen Methoden wie zum Beispiel durch den Zusatz von Säure. Danach wird das auf diese Weise erhaltene Produkt gereinigt, vorzugsweise durch HPLC.

Enthalten die so hergestellten erfindungsgemäßen Peptide eine intramolekulare Disulfidbrücke, so wird die Bildung der Disulfidbrücke vor der Freisetzung des Peptids durch Oxidation an der Festphase mit Jod in Hexafluoroisopropanol/Dichlormethan durchgeführt (Seidel C., in Giralt and Andreu, Eds., Peptides 1991, Escom, Leiden, S. 236)

Es ist also möglich, zur Herstellung der erfindungsgemäßen Peptide, die im wesentlichen aus D-Aminosäuren bestehen, ein standardisiertes Synthese- und Aufreinigungsverfahren einzusetzen. Komplizierte Apparaturen oder aufwendiges Aufreinigen der Entstörpeptide wird vermieden.

Die Peptide, die im wesentlichen aus D-Aminosäuren bestehen, können in monomerer Form eingesetzt werden. Das heißt, jedes Peptid enthält einmal das zu entstörende Epitop in Form von D-Aminosäuren. Erfindungsgemäß können zur Entstörung mehrere verschiedene Peptide mit verschiedenen Epitopen, die im wesentlichen aus D-Aminosäuren bestehen, eingesetzt werden. Dies kann dann zweckmäßig sein, wenn ein Analyt mehrere immunologisch erkennbare Epitope besitzt oder wenn in einem Test verschiedene Analyten detektiert werden.

Die zur Entstörung eingesetzten erfindungsgemäßen Peptide können jedoch das entsprechende Epitop auch mehrfach, also multimer tragen. Sie können also auch als Polyhaptene eingesetzt werden. Dies bedeutet, daß die erfindungsgemäßen Peptide zur Erzeugung eines Polyhaptens, dessen Komponenten im wesentlichen aus D-Aminosäuren bestehen, mehrfach auf einen Träger gekoppelt werden. Dabei können auch verschiedene erfindungsgemäße Peptide, die im wesentlichen aus D-Aminosäuren bestehen, an einen Träger gekoppelt werden. Als Träger kann ein selber nicht an der immunologischen Reaktion teilnehmendes Makromolekül, beispielsweise ein größeres Protein wie Rinderserumalbumin, Partikel aus Latex, Polystyrol, Gold oder Dextran dienen. Die Herstellung von Polyhaptenen kann analog zu der in der WO 96/03652 beschriebenen Methode erfolgen. Anstatt der dort beschriebenen L-Aminosäuren werden für die Herstellung der erfindungsgemäßen Polyhaptene dann D-Aminosäuren verwendet.

Überraschenderweise hat sich gezeigt, daß durch die Verwendung solcher Polyhaptene, bei denen die Peptide, die im wesentlichen aus D-Aminosäuren bestehen, auf ein Trägermaterial gekoppelt sind, eine weitgehende Vermeidung falsch-positiver Reaktionen in Immunoassays möglich ist. Bevorzugt werden die erfindungsgemäßen Peptide daher in Form von Polyhaptenen zur Entstörung von Immunoassays eingesetzt. Die entstörende Wirkung nimmt mit steigender Epitop-Dichte zu. Das heißt, der Entstöreffekt steigt von monomerem Peptid zum Polyhapten.

Die erfindungsgemäßen Peptide, die im wesentlichen aus D-Aminosäuren bestehen, werden als Entstörmittel bei Immunoassays zum Testansatz gegeben. Dabei werden unspezifische Bindungen und Reaktionen, wie sie bei immunologischen Tests wie beispielsweise bei Antikörper-Nachweisen durch die Reaktion der unspezifischen Antikörper mit den Nachweis-Antigenen auftreten, weitestgehend vermieden. Eine mögliche Erklärung für die entstörende Wirkung der erfindungsgemäßen Peptide kann sein, daß die Entstör-Peptide zwar an die unspezifischen Antikörper, nicht jedoch an die spezifischen nachzuweisenden Antikörper binden. Den Entstör-Peptiden, die im wesentlichen aus D-Aminosäuren bestehen, fehlt ja die eigentliche Antigenität. Sie sind offensichtlich gerade deshalb in der Lage, entstörend zu wirken. Es ist möglich, daß die "falsche", unspezifische Reaktion der störenden unspezifischen Antikörper durch Bindung mittels ihrer Antigenbindungsstelle, dem Paratop, an das als Nachweisreagenz eingesetzte Antigen erfolgt. Es kann aber auch eine andere Stelle als das Paratop des Antikörpers mit den Antigenen reagieren. Die so mit dem Entstörmittel blockierten Antikörper können dann nicht mehr mit den Nachweis-Antigenen reagieren, was zur Folge hat, daß weniger oder im Idealfall keine falsch-positiven Ergebnisse mehr auftreten.

Einige Störsubstanzen haben die Eigenschaft, direkt an die Festphase zu binden. Wird nun das erfindungsgemäße Enstörpeptid eingesetzt, kann dieses die Bindungsstellen der Störsubstanz praktisch zukleben und entzieht den Störer damit der immunologischen Reaktion.

In einigen Immuntests treten auch falsch negative Testergebnisse auf. Dies kann darauf zurückzuführen sein, daß ein störender Antikörper in der Probe den nachzuweisenden Antikörper so bindet, daß dessen Antigenbindungsstelle maskiert ist. Somit wird bei Antikörpernachweisen dieser Probenantikörper der eigentlichen Immunreaktion entzogen, und es entsteht ein falsch negatives Resultat. Ein Entstörmittel hat in einem solchen Fall die Aufgabe, den störenden Antikörper oder die störende Substanz so zu binden, daß diese maskiert wird. Die Verwendung der erfindungsgemäßen Peptide, die im wesentlichen aus D-Aminosäuren bestehen, zur Vermeidung falsch-negativer Testergebnisse ist ebenfalls ein Gegenstand der vorliegenden Erfindung.

Die erfindungsgemäßen Peptide, die im wesentlichen aus D-Aminosäuren bestehen, werden bevorzugt im Überschuß zu der in der Probe vorhandenen Störsubstanz verwendet. Eine obere Grenze für die Verwendung der erfindungsgemäßen Peptide besteht nur insofern, als die Löslichkeit des Entstörmittels im Testansatz gewährleistet sein muß. Die Menge bzw. die Konzentration der zur Entstörung eingesetzten Peptide, die im wesentlichen aus D-Aminosäuren bestehen, bzw. der entsprechenden Polyhaptene hängt von der Störsubstanz ab, die sich in einer Probe befindet. Das heißt, je nach Ausmaß der Störung muß eine Menge an Entstörreagenz dazugegeben werden, die vom Fachmann je nach Testführung individuell ermittelt werden muß. Als sinnvoller Konzentrationsbereich hat sich ein Bereich von 1 nmol/l bis 1 mol/l erwiesen.

Erfindungsgemäß und bevorzugt wird durch die Verwendung der erfindungsgemäßen Peptide die Sensitivität des Testes nicht beeinflußt.

Durch die erfindungsgemäßen Peptide, die im wesentlichen aus D-Aminosäuren bestehen, können prinzipiell alle dem Fachmann geläufigen Immunoassay-Formate entstört werden, so daß falsche, insbesondere falsch-positive Testergebnisse weitgehend vermieden werden.

Die erfindungsgemäßen Peptide können in allen Testformaten verwendet werden, sofern ein immunologisch aktiver Bindepartner im Testansatz, das heißt in der Probe oder in den Nachweisreagenzien vorhanden ist. Die erfindungsgemäßen Peptide werden in heterogenen oder homogenen, bevorzugt jedoch in heterogenen Verfahren verwendet.

Bei heterogenen Verfahren ist immer eine Festphase beteiligt, an die der gebildete Komplex aus Analyt und dem oder den Bindepartnern bindet. Beispielhaft ist hier zu nennen der Antikörpernachweis im Brückentest-Format, wie er in der EP-A-280 211 beschrieben ist. Hier wird ein erster Bindepartner, der mit dem zu bestimmenden Antikörper spezifisch bindefähig ist wie beispielsweise ein Antigen, an eine Festphase gebunden. Der zu bestimmende Analyt-Antikörper bindet an das festphasengebundene Antigen. Im Testansatz ist außerdem ein weiteres, für den Analyten spezifischer Bindepartner (Antigen) vorhanden, der mit einer Markierung versehen ist. Sobald die Brücke aus festphasen-gebundenem Bindepartner, Analyt-Antikörper und markiertem Bindepartner gebildet ist, wird die feste von der flüssigen Phase getrennt und die Markierung in der festen oder der flüssigen Phase detektiert. Die erfindungsgemäßen Peptide, die im wesentlichen aus D-Aminosäuren bestehen, bewirken bei diesem Format, daß unspezifische Substanzen in der Probe wie zum Beispiel unspezifische Antikörper so maskiert werden, daß sie nicht mehr an der Bildung des festphasengebundenen Immunkomplexes teilnehmen können. Somit werden insbesondere falsch-positive Testergebnisse weitestgehend vermieden.

Ein weiteres Format, das erfindungsgemäß entstört werden kann, ist die kompetitive Testführung, bei der ein festphasengebundener Komplex aus zwei füreinander spezifischen Bindepartnern gebildet wird, wobei der Bindepartner, der nicht direkt an die Festphase gekoppelt ist, markiert ist. Der Analyt, je nach Testanforderung ein Antigen oder ein Antikörper, verdrängt je nach Konzentration den markierten Bindepartner aus dem Komplex. Nach Trennung der festen von der flüssigen Phase wird die Markierung in einer der Phasen nachgewiesen. Die erfindungsgemäßen Peptide blockieren auch hier die unspezifische Bindung von störenden Probensubstanzen an die als Nachweisreagenzien eingesetzten Bindepartner und verhindern falsche Testergebnisse weit-gehend.

Weitere Formate, die erfindungsgemäß entstört werden, sind der klassische Antigennachweis im Sandwich-Format, bei dem der Analyt (hier ein Antigen) zwischen einem festphasengebundenen und einem markierten Antikörper sandwichartig gebunden wird, sowie der indirekte Nachweis eines Analyt-Antikörpers über dessen Bindung an ein festphasengebundenes Antigen. Der Nachweis erfolgt hier durch die Bindung eines weiteren, markierten Antikörpers an den Analyt-Antikörper.

Selbstverständlich können auch kombinierte Testformate, bei denen gleichzeitig beispielsweise ein Antigen eines Virus und ein gegen ein virales Protein des Virus gerichteter Antikörper nachgewiesen wird, erfindungsgemäß entstört werden.

Auch die Entstörung von homogenen Testformaten ist denkbar. Bei homogenen Testführungen findet im allgemeinen eine Vernetzung von spezifischen Bindepartnern (Antikörpern oder Antigenen) mit einem Analyten statt, die nur in Anwesenheit des Analyten erfolgt. Alternativ können auch bereits vorvernetzte Antigen-Antikörperkomplexe durch Zugabe des Analyten wieder zerstört werden, da der Analyt mit dem Antigen (ein Analyt-Analogon) oder dem Antikörper kompetiert. In jedem Fall wird eine Trübungsmessung bzw. die Änderung der Trübungsdichte nach Analytzugabe durchgeführt. Eine störende Substanz, die anstatt der wahren Analyt-Antikörper die angebotenen Haptene oder Antigene miteinander vernetzt, täuscht eine falsch-positive Reaktion vor. Durch die Verwendung der erfindungsgemäßen Peptide, die im wesentlichen aus D-Aminosäuren bestehen, ist auch hier die weitgehende Vermeidung falsch-positiver Ergebnisse möglich.

Die genannten Testformate, sowie der Nachweis der Analyten sind dem Fachmann geläufig und bedürfen hier keiner weiteren Erläuterung.

Ebenfalls ein Gegenstand der Erfindung ist auch ein immunologisches Verfahren zum Nachweis eines Analyten in einer Probe in einem bekannten Testformat, das dadurch gekennzeichnet ist, daß zur Entstörung Peptide, die im wesentlichen aus D-Aminosäuren bestehen, dem Reaktionsansatz zugesetzt werden. Im allgemeinen wird bei dem erfindungsgemäßen Verfahren die Probe mit einem oder mehreren, für den Analyten spezifischen Bindepartnern und den erfindungsgemäßen Peptiden zur Entstörung in Kontakt gebracht. Dabei kann die Probe vor oder gleichzeitig mit der Zugabe der spezifischen Bindepartner mit den zur Entstörung eingesetzten Peptiden in Kontakt gebracht werden. Anschließend wird der gebildete Komplex aus Analyt und spezifischem Bindepartner als ein Maß für die Anwesenheit des Analyten bestimmt.

Die Testformate zur Durchführung des immunologischen Verfahrens zum Nachweis eines Analyten sind in einem oberen Abschnitt beispielhaft näher erläutert und dem Fachmann überdies als allgemeines Fachwissen bekannt.

Als Analyt können alle Substanzen dienen, die mit mindestens einem spezifischen Bindepartner spezifisch zu einem Komplex reagieren wie zum Beispiel Haptene, Antigene, Antikörper oder Nukleinsäuren.

Als Proben für die Durchführung von den erfindungsgemäß zu entstörenden Immunoassays können alle dem Fachmann geläufigen biologischen Flüssigkeiten verwendet werden. Bevorzugt werden als Probe Körperflüssigkeiten wie Vollblut, Blutserum, Blutplasma, Urin oder Speichel eingesetzt.

Neben den sogenannten Naßtesten, bei denen die Testreagenzien in flüssiger Phase vorliegen, können auch alle gängigen Trockentestformate, die zum immunologischen Nachweis von Analyten geeignet sind, verwendet werden. Bei diesen Trockentests oder Teststreifen, wie sie beispielsweise in der EP-A-0 186 799 beschrieben sind, sind die Testkomponenten auf einem Träger aufgebracht. Die erfindungsgemäßen Peptide, die im wesentlichen aus D-Aminosäuren bestehen, sind dann bereits vor der immunologischen auf dem trockenen Teststreifen aufgebracht.

Das erfindungsgemäße Entstörmittel sollte bei Naßtesten bevorzugt bereits zur Probe gegeben werden, bevor die als Nachweisreagenzien eingesetzten Bindepartner dazugegeben werden, damit die störenden, unspezifischen Substanzen mit dem Entstörmittel reagieren, d.h. an dieses binden können. Als sinnvoll hat es sich erwiesen, die erfindungsgemäßen, im wesentlichen aus D-Aminosäuren bestehenden Peptide bereits im Probenpuffer einzusetzen, der zur Verdünnung der Proben verwendet wird. Die erfindungsgemäßen Peptide werden bevorzugt auch zu den Nachweisreagenzien gegeben.

Ebenfalls Gegenstand der Erfindung ist ein Verfahren zur Entstörung von diagnostischen Nachweismethoden, das dadurch gekennzeichnet ist, daß Peptide, die im wesentlichen aus D-Aminosäuren bestehen, dem Reaktionsansatz zugesetzt werden. Zu den bevorzugten Verfahren, die erfindungsgemäß entstört werden, gehören immundiagnostische Nachweisverfahren von Infektionskrankheiten, insbesondere viralen Ursprungs. Dazu gehören unter anderem Nachweise von Anti-HIV-Antikörpern, HIV-Antigenen, kombinierte Anti-HIV/HIV-Antigen-Nachweise, Anti-HCV-Antikörpern, HCV-Antigenen, kombinierte Anti-HCV/HCV-Antigen-Nachweise.

Ein weiterer Gegenstand der Erfindung ist ein Entstörreagenz, enthaltend mindestens ein Peptid, das im wesentlichen aus D-Aminosäuren besteht. Als weitere Bestandteile des Entstörreagenzes sind dem Fachmann geläufige Puffer, Salze und Detergenzien möglich. Das Entstörreagenz kann in flüssiger, wässriger Form oder in lyophilisierter Form bereitgestellt werden.

Die folgenden Beispiele erläutern die Erfindung weiter.

### Beispiel 1

### Herstellung eines Entstörreagenzes (I) auf Peptidbasis

Das D-Peptid zur Herstellung des Entstörreagenzes wurde mittels Fluorenylmethyloxycarbonyl-(Fmoc)-Festphasenpeptidsynthese an einem Batchpeptidsynthesizer ABI A413 hergestellt. Dazu wurden jeweils 4.0 Äquivalente der in der Tabelle 1 dargestellten Aminosäurederivate 1. bis 16. (BACHEM Bioscience, Heidelberg) verwendet:

**Tabelle 1:**

| | |
|---|---|
| 1. | Fmoc-D-Val-OH |
| 2. | Fmoc-D-Ala-OH |
| 3. | Fmoc-D-Thr (-OtBu)-OH |
| 4. | Fmoc-D-Thr (-OtBu)-OH |
| 5. | Fmoc-D-Cys (Trt)-OH |
| 6. | Fmoc-D-Ile-OH |
| 7. | Fmoc-D-Leu-OH |
| 8. | Fmoc-D-Lys (-Boc)-OH |
| 9. | Fmoc-Gly-OH |
| 10. | Fmoc-D-Ser (Boc)-OH |
| 11. | Fmoc-D-Cys (Trt)-OH |
| 12. | Fmoc-Gly-OH |
| 13. | Fmoc-D-Trp (-Boc)-OH |
| 14. | Fmoc-D-Iso-OH |
| 15. | Fmoc-Gly-OH |
| 16. | Fmoc-D-Leu-OH |
| 17. | Fmoc-β-Ala-OH |
| 18. | Fmoc-ε-Aminocapronsäure |
| 19. | Fmoc-β-Alanin |
| 20. | Boc-L-Lys (-Fmoc)-OH |
| 21. | Fmoc-L-Cys (-Trt)-OH |

Die D-Aminosäurederivate wurden in N-Methylpyrrolidon gelöst. Das D-Peptid wurde an 400 mg 4-(2',4'-Dimethoxyphenyl-Fmoc-Aminomethyl)-Phenoxy-Harz (Tetrahedron Letters 28, 1987, S. 2107) mit einer Beladung von 0.47 mmol/g aufgebaut (JACS 95, 1973, S. 1328). Die Kupplungsreaktionen wurden mit je 4 Äquivalenten N-Hydroxybenzotrialzol und Dicyclohexylcarbodiimid in Dimethylformamid als Reaktionsmedium während 20 min durchgeführt. Nach jedem Kupplungsschritt wurde die Fmoc-Gruppe mittels 20%igem Piperidin in Dimethylformamid in 20 min. abgespalten.

Vor Freisetzung des Peptids wird die Bildung der Disulfidbrücke durch Oxidation an der Festphase mit Jod in Hexafluorisopropanol/Dichlormethan (Seidel C., in Giralt E. and Andreu D. (Eds.) Peptides 1991, Escom, Leiden p. 236) durchgeführt.

Die Freisetzung des Peptids vom Harz und die Abspaltung der permanenten Schutzgruppen erfolgte mittels 20 ml Trifluoressigsäure, 0.5 ml Ethandithiol, 1 ml Thioanisol, 1.5 ml Phenol und 1 ml Wasser in 40 min bei Raumtemperatur. Die Reaktionslösung wurde anschließend mit 300 ml eiskaltem Diisopropylether versetzt und zur vollständigen Fällung des Peptids 40 min bei 0°C gehalten. Der Niederschlag wurde abfiltriert, mit Diisopropylether nachgewaschen, mit wenig 50%iger Essigsäure gelöst und lyophilisiert. Das erhaltene Rohpeptid wurde mittels präparativer HPLC an RP 18-Säulenmaterial mittels eines Gradienten von Acetonitril/Wasser mit 0.1% Trifluoressigsäure in ca. 120 min. aufgereinigt. Die Identität des eluierten Reinmaterials wurde mittels Ionenspray-Massenspektrometrie geprüft.

### Beispiel 2

### Herstellung eines Entstörreagenzes (II) und (III) auf Basis eines Polypeptidkonjugates

Ein kupplungsfähiges D-Peptid, genannt HIV, gp41P2(D)-Cys(1) wird erzeugt, indem zusätzlich noch ein Spacer wie z.B. längere kettenförmige ω-Alkylaminosäuren und ein Cystein N-terminal mittels festphasenpeptidchemischen Methoden vor Freisetzung des Peptids vom Harz angefügt wird. Es kommen dabei die Aminosäuren 17. bis 21. der Tabelle 1 zum Einsatz. Die Oxidation erfolgt jedoch vor der Einführung der Spaceraminosäuren und des Cysteins.

Zur Herstellung des Konjugats wurde zunächst Rinderserumalbumin mit der 12fachen molaren Menge an N-Maleinimidohexanoyl-N-hydroxysuccinimid (MHS) umgesetzt. Die Umsetzung erfolgte in 0.1 mol/l Kaliumphosphatpuffer pH 7.0 und einer Proteinkonzentration von 10 mg/ml innerhalb von 120 min. Die niedermolekularen Bestandteile der Umsetzung wurden mittels Gelpermeationschromatographie (AcA 202-Gel, Biorad) abgetrennt. Dadurch wurden etwa 6 der primären Aminogruppen der Lysinseitenketten mit Maleinimidogruppen modifiziert.

Das D-Peptid wurde mit dem maleinimido-funktionalisierten Rinderserumalbumin in 0.1 mol/l Kaliumphosphatpuffer pH 7.0 innerhalb von 120 min umgesetzt. Die Abtrennung von nicht umgesetztem Peptid und die Separation von monomerem und polymerem Konjugat erfolgte auf Sephacryl-S200 HR. Bei einer typischen Umsetzung in 20.8 ml Puffer von 206 mg aktiviertem Protein mit 102 mg HIV, gp41P2(D)-Cys Peptid (I) werden 27.7 mg polymeres (II) und 76.3 mg monomeres gelöstes Proteinkonjugat (III) erhalten. Die Lösung wird mit Trehalose im 40fachen Verhältnis und lyophilisiert.

### Beispiel 3

### Entstörung eines Immunoassays zum Nachweis von Anti-HIV-Antikörpern im Microspot®-Format

Microspot® ist eine miniaturisierte, ultrasensitive Technologie, die ideal zur gleichzeitigen Bestimmung von verschiedenen diagnostischen Parametern in einem einzigen Meßvorgang geeignet ist. Die hier zugrundeliegende Technologie wird beispielsweise im US-Patent 5,599,729 beschrieben.

Der hier beispielhaft beschriebene Test wird im sogenannten Brückentestformat durchgeführt, bei dem zwei Antigene den nachzuweisenden Probenantikörper miteinander verbrücken. Im Falle der Bestimmung von Anti-HIV-Antikörpern (auch als <HIV>-Antikörper bezeichnet) sind die einzelnen HIV-Antigene in sogenannten "Arrays" auf einem Polystyrolträger immobilisiert. Die einzelnen HIV-Antigene werden dabei als Spots mittels einer zur Inkjet-Technologie verwandten Technologie auf das Testfeld aufgebracht. Bei der Testdurchführung werden auf die Testfläche 30 µl mit Probenpuffer vorverdünnte Probe (beispielsweise Blutserum) pipettiert und 20 Minuten unter Schütteln bei Raumtemperatur inkubiert. Nach Absaugen der Probe und Waschen des Testfeldes mit Waschpuffer werden 30µl Reagenzlösung 1, die eine Mischung aller Digoxigenin-markierten HIV-Antigene enthält, auf das Testfeld pipettiert und wiederum 20 Minuten unter Schütteln bei Raumtemperatur inkubiert. Die Sequenzen der immobilisierten Antigene entsprechen den Sequenzen der in Reagenzlösung 1 vorhandenen Digoxigenin-markierten HIV-Antigene. Nach Absaugen der Reagenzlösung 1 und Waschen des Testfeldes mit Waschpuffer werden 30µl Reagenzlösung 2, die das Nachweisreagenz enthält, auf das Testfeld pipettiert. Als Nachweisreagenz dienen 100 nm große, fluoreszenzgefärbte Latexpartikel, die kovalent mit einem Anti-Digoxigenin-Antikörper beschichtet sind.

Dieses Nachweisreagenz wird wiederum 20 Minuten unter Schütteln bei Raumtemperatur inkubiert, anschließend abgesaugt, gewaschen und trockengesaugt. Das Testfeld wird mit einem He-Ne-Laser mit 633 nm Wellenlänge bestrahlt und die Fluoreszenz bei 670 nm Wellenlänge mit einer CCD-Kamera vermessen.

### Probenpuffer:

50 mM Tris pH 7.6
0.05 % Tween 20
0.5% RSA
0.1% R-IgG
0.01% MIT

### Reagenzlösung 1:

(Boehringer Mannheim GmbH, Id.Nr. 1650807, Enzymun <HCV> Inkubationspuffer)
50 mM Na-Phosphat-Puffer
120 mM NaCl
0.01% MIT
20% PDB

Im Microspot® <HIV>-Assay werden zwei verschiedene Peptide, die zwei unterschiedliche Epitope des gp41-Antigens repräsentieren, aufgebracht Beim Screenen von ca. 500 negativen Proben wurden 4 Proben als falsch positiv detektiert. Dabei fiel auf, daß alle 4 Proben mit dem gp41-Peptid 2-Antigenspot unspezifisch reagieren, während alle anderen Spots keine Reaktion zeigen. Trotz aller durchgeführten Optimierungsmaßnahmen wie Rohstoff- und Pufferoptimierungen konnte keine Verbesserung der Spezifität erreicht werden. Erst durch die Zugabe eines sequenzgleichen Peptids, das aus D-Aminosäuren synthetisiert wurde, konnte eine vollständige Entstörung bei 3 der 4 Proben erzielt werden. Der beste entstörende Effekt konnte bei Verwendung eines polymeren Entstörmoleküls (II), d.h. einem Polyhapten, welches 6 HIV, gp41P2(D) Moleküle pro RSA-Trägermolekül enthält, erreicht werden (siehe Tabellen 1 und 2):

**Tabelle 1:**

| **Ergebnisse mit Proben- und Reagenz-1-Puffer ohne Entstörreagenz auf dem gp41-Peptid 2-Spot:** | | | | |
|---|---|---|---|---|
| Probe | Untergrund* [Counts] | Signal gp41-P2-[Counts] | Signal gp41-P2-Untergrund | Cut-off-Index** |
| Negativkontrolle | 33 | 33 | 0 | **0.0** |
| Positivkontrolle | 57 | 2642 | 2585 | **39.2** |
| Positiv-Probe 1 | 145 | 26685 | 26540 | **402.1** |
| Positiv-Probe 2 | 55 | 3158 | 3103 | **47.0** |
| Störprobe 1 | 43 | 980 | 937 | **14.2** |
| Störprobe 2 | 28 | 767 | 739 | **11.2** |
| Störprobe 3 | 35 | 877 | 842 | **12.8** |
| Störprobe 4 | 37 | 204 | 167 | **2.5** |
| Negativprobe | 29 | 29 | 0 | **0.0** |

| | | | | |
|---|---|---|---|---|
| * Untergrund entspricht Signal neben den Testfeldern | | | | |
| ** Cut-off-Index = Signal_{Probe}-Signal_{Untergrund}/2xSignal_{Negativkontrolle} | | | | |

**Tabelle 2:**

| **Ergebnisse mit Entstörreagenz (100 µg/ml im Probenpuffer und 10 µg/ml im Reagenz-1-Puffer) auf dem gp41-Peptid 2-Spot:** | | | | |
|---|---|---|---|---|
| Probe | Untergrund* [Counts] | Signal gp41-P2 [Counts] | Signal gp41-P2-Untergrund | Cut-off-Index** |
| Negativkontrolle | 28 | 28 | 0 | **0.0** |
| Positivkontrolle | 53 | 2258 | 2205 | **39.4** |
| Positiv-Probe 1 | 115 | 25951 | 25836 | **461.4** |
| Positiv-Probe 2 | 57 | 2650 | 2593 | **46.3** |
| Störprobe 1 | 42 | 152 | 110 | **2.0** |
| Störprobe 2 | 26 | 26 | 0 | **0.0** |
| Störprobe 3 | 30 | 30 | 0 | **0.0** |
| Störprobe 4 | 34 | 34 | 0 | **0.0** |

| | | | | |
|---|---|---|---|---|
| * Untergrund entspricht Signal neben des Testfeldern | | | | |
| ** Cut-off-Index = Signal_{Probe} - Signal_{Untergrund}/2 x Signal_{Negativkontrolle} | | | | |

Dieses Ergebnis zeigt, daß der Microspot® <HIV>-Test durch 4 Proben stark gestört wird und diese ein falsch positives Ergebnis erzeugen. Durch den Zusatz des erfindungsgemäßen Entstörreagenzes zu Proben- und Reagenz-1-Puffer können 3 der 4 Proben eindeutig entstört werden, während das Störsignal der 4. Probe deutlich reduziert wird. Überraschend ist die Tatsache, daß die Signale der positiven Proben nicht oder nur minimal reduziert werden, so daß die Sensitivität des Tests vollständig erhalten bleibt.

## Patentansprüche

1. Verwendung von Peptiden, die im wesentlichen aus D-Aminosäuren bestehen, zur Entstörung von immundiagnostischen Verfahren.

2. Verwendung von Peptiden gemäß Anspruch 1 **dadurch gekennzeichnet, daß** die Peptide als Polyhapten vorliegen.

3. Verwendung von Peptiden gemäß Anspruch 1 oder 2 zur Entstörung von heterogenen Immunoassays.

4. Verwendung von Peptiden gemäß Anspruch 3 zur Entstörung von Immunoassays nach dem Prinzip des Brückentests.

5. Verwendung von Peptiden gemäß Anspruch 3 zur Entstörung von Immunoassays nach dem Sandwichprinzip.

6. Verwendung von Peptiden gemäß Anspruch 3 zur Entstörung von Immunoassays nach dem indirekten Testprinzip.

7. Verwendung von Peptiden gemäß Anspruch 3 zur Entstörung von kompetitiven Immunoassays.

8. Verwendung von Peptiden gemäß Anspruch 1 oder 2 zur Entstörung von homogenen Immunoassays.

9. Immunologisches Verfahren zum Nachweis eines Analyten, umfassend die Schritte: Kontaktieren der Probe mit Peptiden die im wesentlichen aus D-Aminosaüren bestehen, Kontaktieren der Probe mit einem oder mehreren spezifischen Bindepartnern des Analyten, Messung des gebildeten Komplexes aus Analyt und spezifischem Bindepartner als ein Maß für die Anwesenheit des Analyten.

10. Verfahren zur Entstörung von diagnostischen Nachweismethoden, **dadurch gekennzeichnet, daß** Peptide, die im wesentlichen aus D-Aminosäuren bestehen, dem Reaktionsansatz zugesetzt werden.

## Claims

1. Use of peptides which are essentially composed of D-amino acids to eliminate interference of immunodiagnostic methods.

2. Use of peptides as claimed in claim 1, **characterized in that** the peptides are present as a polyhapten.

3. Use of peptides as claimed in claim 1 or 2 to eliminate interference of heterogeneous immunoassays.

4. Use of peptides as claimed in claim 3 to eliminate interference of immunoassays based on the bridge test principle.

5. Use of peptides as claimed in claim 3 to eliminate interference of immunoassays based on the sandwich principle.

6. Use of peptides as claimed in claim 3 to eliminate interference of immunoassays based on the indirect test principle.

7. Use of peptides as claimed in claim 3 to eliminate interference of competitive immunoassays.

8. Use of peptides as claimed in claim 1 or 2 to eliminate interference of homogeneous immunoassays.

9. Immunological method for the detection of an analyte comprising the steps: contacting the sample with peptides which are essentially composed of D-amino acids, contacting the sample with one or several specific binding partner(s) of the analyte, measuring the complex formed of analyte and specific binding partner as a measure for the presence of the analyte.

10. Method for eliminating interference of diagnostic detection methods, **characterized in that** peptides which are essentially composed of D-amino acids are added to the reaction mixture.

## Revendications

1. Utilisation de peptides, qui sont constitués pour l'essentiel d'acides D-aminés, pour réduire les interférences dans les procédés d'immunodiagnostic.

2. Utilisation de peptides selon la revendication 1, **caractérisée en ce que** les peptides existent sous forme de polyhaptène.

3. Utilisation de peptides selon la revendication 1 ou 2, pour réduire les interférences dans les immunodosages hétérogènes.

4. Utilisation de peptides selon la revendication 3, pour réduire les interférences dans les immunodosages selon le principe du test à pont.

5. Utilisation de peptides selon la revendication 3, pour réduire les interférences dans les immunodosages selon le principe du sandwich.

6. Utilisation de peptides selon la revendication 3, pour réduire les interférences dans les immunodosages selon le principe de test indirect.

7. Utilisation de peptides selon la revendication 3, pour réduire les interférences dans les immunodosages compétitifs.

8. Utilisation de peptides selon la revendication 1 ou 2, pour réduire les interférences dans les immunodosages homogènes.

9. Procédé immunologique pour détecter un analyte, comprenant les étapes : la mise en contact de l'échantillon avec des peptides qui sont constitués essentiellement d'acides D-aminés, la mise en contact de l'échantillon avec un ou plusieurs partenaires de liaison spécifiques de l'analyte, la mesure du complexe formé à partir de l'analyte et du partenaire de liaison spécifique en tant que mesure de la présence de l'analyte.

10. Procédé pour réduire les interférences dans les procédés de détection pour diagnostic, **caractérisé en ce que** des peptides qui sont constitués pour l'essentiel d'acides D-aminés, sont ajoutés au mélange réactionnel.
